# FASCICULE DE BREVET EUROPEEN

(11) **EP 1 104 665 B1**
(45) Date de publication et mention de la délivrance du brevet: **31.03.2004**
(21) Numéro de dépôt: 99402857.9
(22) Date de dépôt: 18.11.1999
(51) Int. Cl.: A61F 2/44

(54) **Cage intervertébrale**
Zwischenwirbelkäfig
Intervertebral cage

(43) Date de publication de la demande: 06.06.2001
(73) Titulaire: Razian, Hassan, 94800 Villejuif (FR)
(72) Inventeur: Razian, Hassan, 94800 Villejuif (FR); Herman, Serge, 75005 Paris (FR); Robine, Dominique, 75008 Paris (FR)
(74) Mandataire: Flavenot, Bernard

(56) Documents cités:
- EP-A- 0 646 366
- WO-A-95/08306
- WO-A-96/27348
- WO-A-97/06753
- DE-C- 4 327 054
- US-A- 5 683 394

## Description

La présente invention concerne les cages intervertébrales trouvant une application particulièrement avantageuse, mais non exclusivement, pour le traitement du rachis dégénératif.

Une cage intervertébrale, notamment pour le traitement du rachis dégénératif, est déjà connue. Elle comporte essentiellement une entretoise en forme de disque comprenant deux faces de base opposées sensiblement planes et parallèles et une paroi latérale reliant les deux faces de base. Cette entretoise est apte à être disposée entre les faces en regard des deux corps vertébraux, respectivement de deux vertèbres consécutives, en remplacement du disque endommagé situé entre ces deux vertèbres, les deux faces de base de l'entretoise étant placées au contact des corps vertébraux. L'entretoise comporte en outre une cavité ouverte dans laquelle il est possible de placer un greffon osseux ou analogue dans le but de souder entre eux les deux corps vertébraux par ostéosynthèse.

Bien que cette réalisation donne généralement de bons résultats, elle peut, dans des circonstances très exceptionnelles, ne pas permettre à la cage intervertébrale de remplir le rôle pour lequel elle était prévue.

En effet, dans les premiers temps suivant l'implantation de la cage, on constate que, lors de certains mouvements de relativement grande amplitude de la colonne vertébrale, par exemple quand la tête se penche très en arrière, il peut se créer un espace non négligeable entre les faces de base de l'entretoise et les vertèbres, par lequel le greffon ou une partie de celui-ci peut s'échapper de la cavité dans laquelle il était placé. Si c'est le cas, le greffon ne peut plus alors jouer correctement son rôle et une nouvelle intervention chirurgicale est nécessaire.

Pour tenter de pallier les inconvénients mentionnés ci-dessus, il a été conçu des cages comportant une patte d'ancrage pivotant sur une face de l'entretoise. Une telle cage est par exemple décrite dans le US-A-5 683 394. Cette réalisation donne théoriquement de bons résultats mais présente parfois des insuffisances notamment quant au maitien de la patte d'ancrage dans sa position de blocage de l'entretoise.

Aussi la présente invention a-t-elle pour but de réaliser une cage intervertébrale qui pallie les inconvénients mentionnés ci-dessus de la cage intervertébrale décrite schématiquement dans ce préambule, et dont la structure permet en plus un très bon ancrage de la cage dans les vertèbres entre lesquelles elle doit être implantée.

Plus précisément, la présente invention a pour objet une cage intervertébrale, notamment pour le traitement du rachis dégénératif selon la revendication 1.

D'autres caractéristiques et avantages de la présente invention apparaîtront au cours de la description suivante donnée en regard des dessins annexés à titre illustratif, mais nullement limitatif, dans lesquels:

Les figures 1 à 3 représentent un mode de réalisation d'une cage intervertébrale selon l'invention, les figures 1 et 2 représentant deux coupes orthogonales, la coupe selon la figure 1 étant référencée I-I sur la figure 2, la coupe selon la figure 2 étant référencée II-II sur la figure 1, la figure 3 représentant une vue de face selon la flèche III sur les figures 1 et 2, et

La figure 4 représente, en vue de côté, une cage intervertébrale selon les figures 1 à 3 implantée entre deux vertèbres.

Le Demandeur tient à préciser que ces figures ne représentent qu'un exemple de mode de réalisation de l'objet selon l'invention, mais qu'il peut exister d'autres modes de réalisation qui répondent à la définition de cette invention, notamment celle donnée dans la première revendication.

Il précise en outre que, lorsque, selon la définition de l'invention son objet comporte "au moins un" élément ayant une fonction donnée, le mode de réalisation décrit peut comporter plusieurs de ces éléments.

Il précise aussi que, si le mode de réalisation de l'objet selon l'invention tel qu'illustré comporte plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

Les figures représentant un seul mode de réalisation d'une cage intervertébrale selon l'invention, les mêmes références y désignent les mêmes moyens, quelle que soit la forme sous laquelle ils sont représentés.

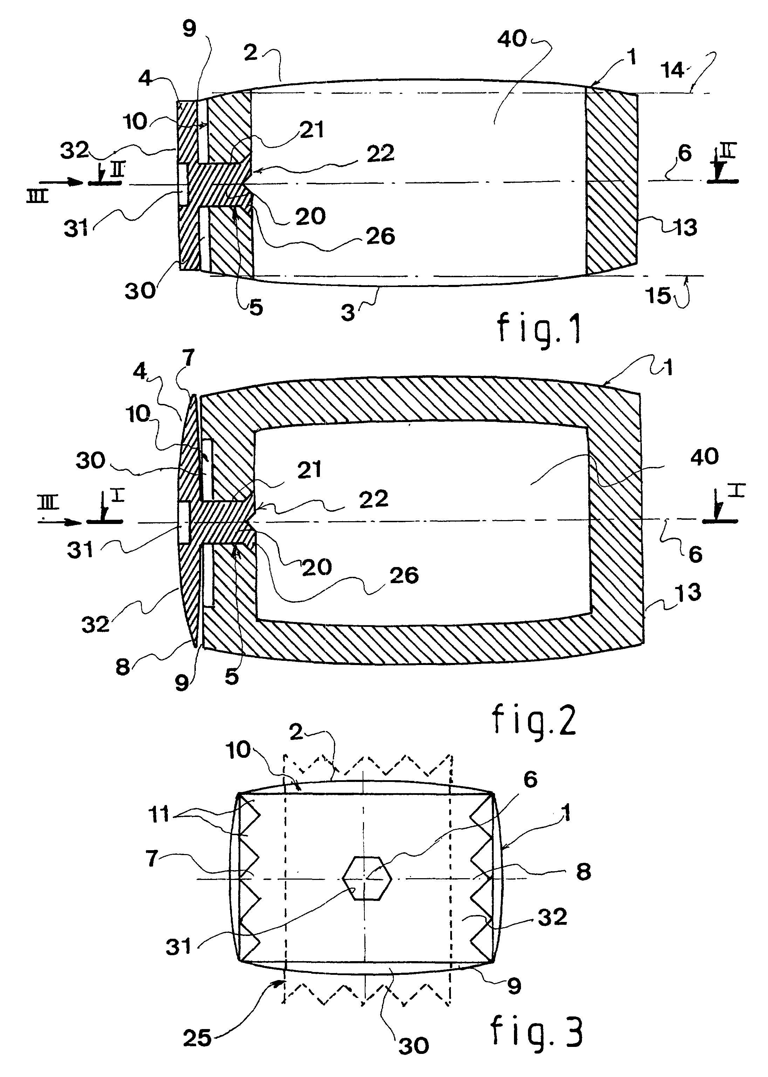

La cage intervertébrale représentée sur les figures 1 à 3 comporte une entretoise 1 en forme de disque comprenant deux faces de base 2, 3 opposées sensiblement planes et parallèles et une paroi latérale 13 reliant ces deux faces de base, une patte 4 et des moyens 5 pour monter cette patte en rotation par rapport à l'entretoise 1 autour d'un axe 6 sensiblement parallèle aux plans des faces de base 2, 3, respectivement représentés en traits interrompus 14, 15 sur la figure 1, la distance séparant au moins une première extrémité 7, 8 de la patte 4 et l'axe de rotation 6 étant supérieure à la distance séparant au moins l'une des deux faces de base 2, 3 et le même axe de rotation.

Quant à la patte, elle est avantageusement conformée de façon qu'au moins sa première extrémité 7, 8 soit en forme de biseau, figures 1 et 2. Cette première extrémité 7, 8 de la patte 4 peut même porter une denture 11, figure 3, comme celle d'une scie ou analogue, en sachant que cette denture peut se combiner ou non avec la forme en biseau.

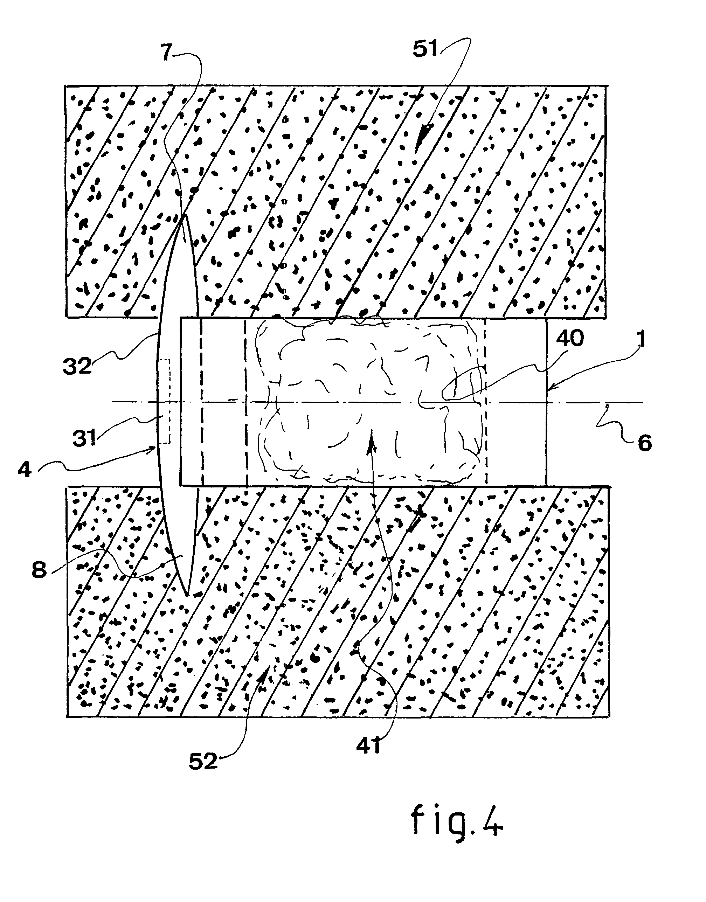

Il est cependant avantageux que la patte soit conformée de façon que les distances séparant respectivement ses deux extrémités 7, 8 et l'axe de rotation 6 soient supérieures aux distances séparant respectivement les deux faces de base 2, 3 et l'axe de rotation 6. Comme il sera décrit ci-après en regard de la figure 4, cette caractéristique permettra aux deux extrémités 7, 8 de la patte 4 de pénétrer respectivement dans les deux vertèbres entre lesquelles la cage est destinée à être implantée.

Il est bien précisé que la forme générale de l'entretoise 1 est définie, pour les besoins des présentes, comme celle d'un disque à deux faces de base sensiblement planes et parallèles, quelle que soit la forme du contour de ce disque, par exemple circulaire, rectangulaire, etc. Il est également précisé que, même si ces faces de base 2, 3 ont été définies comme étant sensiblement planes, elles peuvent présenter, de même que la paroi latérale 13, des formes légèrement arrondies ou bombées, comme représenté sur les figures 1 à 3.

Dans une réalisation préférentielle, la cage intervertébrale comporte en outre des moyens 10 pour bloquer la patte 4, au moins en rotation, quand elle est amenée dans une position sensiblement orthogonale au plan des faces de base 2, 3. Ces moyens comportent avantageusement au moins une rainure 30 réalisée dans la paroi latérale 13 de l'entretoise 1 et débouchant sur les deux faces de base 2, 3. Cette rainure est réalisée dans la paroi latérale 13 de l'entretoise 1 de façon que la patte 4 puisse s'encastrer dans cette rainure quand elle est amenée dans une position sensiblement orthogonale au plan des faces de base 2, 3, la largeur de la rainure étant sensiblement égale à la largeur de la patte. Cette position de la patte est représentée sur la figure 3 en traits interrompus référencés 25.

Dans une réalisation avantageuse, ces moyens 10 comportent, en plus des éléments mentionnés et décrits ci-dessus, des moyens 22 pour appliquer une force élastique sur la patte 4 et tendre à la plaquer contre la paroi latérale 13 de l'entretoise, et donc au fond de la rainure 30 quand elle est dans sa position orthogonale définie ci-dessus. Ces moyens élastiques 22 peuvent être de tous types, par exemple un ressort ou analogue dont les deux extrémités coopèrent respectivement avec l'entretoise 1 et la patte 4 pour les pousser ou les attirer l'une vers l'autre de façon élastique. Un mode de réalisation préférentiel de ces moyens élastiques 22 sera donné ci-après.

Comme mentionné ci-avant, la cage intervertébrale comporte des moyens 5 pour monter la patte 4 en rotation par rapport à l'entretoise 1 autour de l'axe 6. Dans le mode de réalisation illustré, ces moyens 5 comportent essentiellement un arbre de rotation 20 solidaire de la patte 4, un orifice 21 réalisé dans l'entretoise 1 en étant centré sur l'axe de rotation 6, l'arbre 20 étant monté en rotation dans l'orifice 21, et des moyens 26 pour bloquer l'arbre en translation par rapport à l'entretoise 1. Ces moyens de blocage en translation 26 sont par exemple constitués, comme illustré sur les figures 1 et 2, par un sertissage, dans l'orifice 21, de l'extrémité de l'arbre 20 opposée à celle qui est solidaire de la patte 4.

Ces moyens 5 peuvent aussi être constitués par le fait que l'arbre 20 et l'orifice 21 comportent des filetages respectivement complémentaires l'un de l'autre de façon que l'arbre puisse être vissé dans cet orifice pour que la patte 4 puisse subir une rotation par rapport à l'entretoise d'un angle d'une amplitude pouvant aller, par exemple, jusqu'à quatre-vingt-dix degrés. Ce mode de réalisation des moyens 5 n'a pas été spécifiquement illustré.

Quand les moyens 5 pour monter la patte 4 en rotation par rapport à l'entretoise 1 autour de l'axe 6 ont la structure décrite ci-dessus et illustrée, il est avantageux que les moyens 22 pour appliquer une force élastique entre la patte 4 et l'entretoise 1, au lieu d'être constitués par un ressort de type classique, soient constitués par le fait que l'arbre 20 est monté dans l'orifice 21 en traction élastique par rapport à l'entretoise, l'élasticité naturelle du matériau constituant la patte 4 et/ou l'arbre 20 réalisant elle-même la fonction de ces moyens élastiques 22. Cette réalisation est d'autant plus possible que, comme décrit ci-après, la patte 4 n'est en fait soumise qu'une seule fois à des tensions de traction pour l'amener dans la rainure 30.

La cage intervertébrale 1 comporte en outre une cavité 40 ouverte sur au moins l'une des deux faces de base 2, 3, cette cavité ouverte étant apte à contenir un greffon osseux 41 dont la fonction est bien connue en elle-même.

Dans une forme avantageuse comme celle illustrée, le disque présente une forme sensiblement parallélépipédique rectangle. Dans ce cas, la patte 4 est montée rotative sur l'une 9 des deux plus petites faces latérales de l'entretoise 1, l'axe de rotation 6 étant sensiblement perpendiculaire à cette petite face latérale 9. Dans cette réalisation, la patte 4 présente avantageusement une forme sensiblement rectangulaire. Sa largeur et sa longueur sont respectivement sensiblement égales à la largeur et à la longueur de la petite face latérale 9 sur laquelle elle est montée en rotation.

La cage intervertébrale décrite ci-dessus est destinée à remplacer un disque intervertébral endommagé et s'utilise de la façon suivante:

Le disque endommagé ayant été enlevé, la cage est introduite entre les deux corps de vertèbre 51, 52, avec les deux faces de base 2, 3 à leur contact, le greffon osseux ayant été préalablement introduit dans la cavité ouverte 40.

La technique de mise en place de la cage est du domaine du chirurgien. Elle est bien est connue en elle-même et, comme elle n'est pas l'objet de la présente invention, elle ne sera pas décrite ici en détail.

Il est cependant précisé que la mise en place de la cage s'effectue avec la patte 4 dans une position parallèle aux plans des faces de base 2, 3, c'est-à-dire dans une position comme celle qui est illustrée sur les figures 1 à 3 en traits continus. Après quoi, la patte est pivotée autour de l'axe 6 au moyen d'un instrument qui peut se placer dans une fente 31 ou analogue réalisée dans la face 32 de la patte opposée à sa face qui est tournée vers l'entretoise 1, notamment vers la petite face 9 de la paroi latérale 13.

La rotation de la patte est effectuée jusqu'à ce qu'elle vienne dans la position orthogonale définie ci-avant, celle représentée en traits interrompus 25 dans la vue de face sur la figure 3 et en traits pleins dans la vue de côté sur la figure 4. Dans cette position, la patte 4 se loge dans la rainure 30 et y est bloquée en rotation, ainsi qu'en translation sous l'effet de la traction élastique exercée par l'arbre 20 comme mentionné ci-avant.

La cage est ainsi parfaitement maintenue entre les deux corps de vertèbre, ce qui empêche notamment sa migration entre les deux vertèbres dès sa pose et, en outre, par sa largeur, même dans le cas d'une importante amplitude de rotation de l'une des vertèbres par rapport à l'autre comme décrit au préambule de la présente description, la patte 4 empêche le greffon ou une partie de celui-ci de s'échapper de la cavité 40.

Lorsque la cage ne comporte qu'une seule patte 4, elle sera généralement implantée de façon que cette patte soit située vers l'avant de la colonne vertébrale, sauf par exemple dans le cas d'une implantation dans la région lombaire, dans lequel cas la patte pourra avantageusement être située vers l'arrière de la colonne vertébrale.

Il est cependant possible, dans une réalisation avantageuse, que la cage intervertébrale comporte deux pattes montées en coopération avec l'entretoise, par exemple en deux positions opposées sur la paroi latérale 13 de celle-ci.

## Revendications

1. Cage intervertébrale, notamment pour le traitement du rachis dégénératif, comprenant,
- une entretoise (1) en forme de disque comportant deux faces de base (2, 3) opposées sensiblement planes et parallèles et une paroi latérale (13) reliant les deux faces de base,
- au moins une patte (4)
- des moyens (5) pour monter la patte (4) en rotation par rapport à l'entretoise (1) autour d'un axe (6) sensiblement parallèle au plan des faces de base (2, 3), la distance séparant au moins une première extrémité (7, 8) de la patte et l'axe de rotation (6) étant supérieure à la distance séparant au moins l'une des deux faces de base (2, 3) et ledit axe de rotation, lesdits moyens (5) comportant un arbre de rotation (20) solidaire de la patte (4) et un orifice (21) réalisé dans l'entretoise (1) en étant centré sur l'axe de rotation (6), l'arbre (20) étant monté en rotation dans l'orifice, **caractérisée par le fait qu'**elle comporte en outre
- des moyens (26) pour bloquer l'arbre (20) en translation par rapport à l'entretoise (1), et
- des moyens (22) pour appliquer une force élastique sur la patte (4) et tendre à la plaquer contre la paroi latérale de l'entretoise constitués **par le fait que** l'arbre (20) est monté en traction élastique dans l'orifice (21).

2. Cage selon la revendication 1, **caractérisée par le fait que** la première extrémité (7, 8) de la patte (4) est conformée en biseau.

3. Cage selon l'une des revendications 1 et 2, **caractérisée par le fait qu'**elle comporte une fente (31) réalisée dans la face (32) de la patte (4) qui est tournée vers l'entretoise (1).

4. Cage selon l'une des revendications 1 à 3, **caractérisée par le fait qu'**elle comporte des moyens (10) pour bloquer la patte (4) quand elle est amenée dans une position sensiblement orthogonale au plan des faces de base (2, 3).

5. Cage selon la revendication 4, **caractérisée par le fait que** les moyens (10) pour bloquer la patte (4) quand elle est amenée dans une position sensiblement orthogonale au plan des faces de base (2, 3) comportent au moins une rainure (30) réalisée dans la paroi latérale (13) de l'entretoise (1) et débouchant sur les deux faces de base (2, 3), la rainure (30) étant réalisée dans la paroi latérale de l'entretoise de façon à recevoir la patte (4) quand cette dernière est sensiblement orthogonale au plan des faces de bases (2, 3), la largeur de la rainure (30) étant sensiblement égale à la largeur de la patte.

6. Cage selon l'une des revendications 1 à 5, **caractérisée par le fait que** la première extrémité (7, 8) de la patte porte une denture (11).

7. Cage selon l'une des revendications 1 à 6, **caractérisée par le fait que** les distances séparant respectivement les deux extrémités (7, 8) de la patte (4) et l'axe de rotation (6) sont supérieures aux distances séparant respectivement les deux faces de base (2, 3) et ledit axe de rotation.

8. Cage selon l'une des revendications 1 à 7, **caractérisée par le fait que** ladite entretoise (1) comporte une cavité (40) ouverte sur au moins l'une des deux faces de base (2, 3), cette cavité étant apte à contenir un greffon osseux (41).

9. Cage selon l'une des revendications 1 à 8, **caractérisée par le fait que** le disque présente une forme sensiblement parallélépipédique rectangle.

10. Cage selon la revendication 9, **caractérisée par le fait que** la patte (4) est montée rotative sur l'une (9) des deux plus petites faces latérales de l'entretoise (1), l'axe de rotation (6) étant sensiblement perpendiculaire à ladite petite face latérale.

11. Cage selon la revendication 10, **caractérisée par le fait que** la largeur et la longueur de la patte (4) sont respectivement sensiblement égales à la largeur et à la longueur de la petite face latérale (9) sur laquelle elle est montée en rotation.

## Patentansprüche

1. Zwischenwirbelkäfig, insbesondere für die Behandlung der degenerativen Wirbelsäule, umfassend:
- eine Querstrebe (1) in Form einer Scheibe, die zwei im Wesentlichen ebene und parallele gegenüberliegende Grundflächen (2, 3) sowie eine die zwei Grundflächen verbindende Seitenwand (13) aufweist,
- wenigstens eine Klammer (4),
- Mittel (5) für die Anbringung der Klammer (4), derart, dass sie in Bezug auf die Querstrebe (1) um eine zu der Ebene der Grundflächen (2, 3) im Wesentlichen parallele Achse drehbar ist, wobei der Abstand, der wenigstens ein erstes Ende (7, 8) der Klammer von der Drehachse (6) trennt, größer als der Abstand ist, der wenigstens eine der Grundflächen (2, 3) von der Drehachse trennt, wobei die Mittel (5) eine Drehwelle (20), die mit der Klammer (4) fest verbunden ist, und eine Öffnung (21), die in der Querstrebe (1) zentriert auf die Drehachse (6) verwirklicht ist, umfassen, wobei die Welle (20) in der Öffnung drehbar angebracht ist, **dadurch gekennzeichnet, dass** er außerdem umfasst:
- Mittel (26), die die Welle (20) in Bezug auf die Querstrebe (1) translatorisch blockieren, und
- Mittel (22), die auf die Klammer (4) eine elastische Kraft ausüben und bestrebt sind, sie gegen die Seitenwand der Querstrebe zu drängen und dadurch gebildet sind, dass die Welle (20) in der Öffnung (21) unter elastischem Zug angebracht ist.

2. Käfig nach Anspruch 1, **dadurch gekennzeichnet, dass** das erste Ende (7, 8) der Klammer (4) angefast ist.

3. Käfig nach einem der Ansprüche 1 und 2, **dadurch gekennzeichnet, dass** er einen Schlitz (31) aufweist, der in jener Fläche (32) der Klammer (4) verwirklicht ist, die der Querstrebe (1) zugewandt ist.

4. Käfig nach einem der Ansprüche 1 bis 3, **dadurch gekennzeichnet, dass** er Mittel (10) umfasst, die die Klammer (4) blockieren, wenn sie in eine zu der Ebene der Grundflächen (2, 3) im Wesentlichen senkrechte Stellung geführt ist.

5. Käfig nach Anspruch 4, **dadurch gekennzeichnet, dass** die Mittel (10), die die Klammer (4) blockieren, wenn sie in eine zu der Ebene der Grundflächen (2, 3) im Wesentlichen senkrechte Stellung geführt ist, wenigstens eine Nut (30) aufweisen, die in der Seitenwand (13) der Querstrebe verwirklicht ist und in die zwei Grundflächen (2, 3) mündet, wobei die Nut (30) in der Seitenwand der Querstrebe in der Weise verwirklicht ist, dass sie die Klammer (4) aufnimmt, wenn diese letztere zu der Ebene der Grundflächen (2, 3) im Wesentlichen senkrecht ist, wobei die Breite der Nut (30) im Wesentlichen gleich der Breite der Klammer ist.

6. Käfig nach einem der Ansprüche 1 bis 5, **dadurch gekennzeichnet, dass** das erste Ende (7, 8) der Klammer eine Zahnung (11) trägt.

7. Käfig nach einem der Ansprüche 1 bis 6, **dadurch gekennzeichnet, dass** die Abstände, die die beiden Enden (7, 8) der Klammer (4) jeweils von der Drehachse (6) trennen, größer als die Abstände sind, die die beiden Grundflächen (2, 3) jeweils von der Drehachse trennen.

8. Käfig nach einem der Ansprüche 1 bis 7, **dadurch gekennzeichnet, dass** die Querstrebe (1) einen Hohlraum (40) aufweist, der an wenigstens einer der zwei Grundflächen (2, 3) offen ist, wobei dieser Hohlraum ein Knochentransplantat (41) enthalten kann.

9. Käfig nach einem der Ansprüche 1 bis 8, **dadurch gekennzeichnet, dass** die Scheibe eine im Wesentlichen parallelepipedische, rechtwinklige Form hat.

10. Käfig nach Anspruch 9, **dadurch gekennzeichnet, dass** die Klammer (4) an einer (9) der zwei kleineren Seitenflächen der Querstrebe (1) drehbar angebracht ist, wobei die Drehachse (6) zu der kleinen Seitenfläche im Wesentlichen senkrecht ist.

11. Käfig nach Anspruch 10, **dadurch gekennzeichnet, dass** die Breite und die Länge der Klammer (4) im Wesentlichen gleich der Breite bzw. der Länge der kleinen Seitenfläche (9) ist, auf der sie drehbar angebracht ist.

## Claims

1. An intervertebral cage, in particular for treating degeneration of the spine, the cage comprising:
· a spacer (1) in the form of a disk having two opposite base faces (2, 3) that are substantially plane and parallel to each other, and a side wall (13) interconnecting the two base faces;
· at least one tab (4); and
· means (5) for mounting the tab (4) to turn relative to the spacer (1) about an axis (6) substantially parallel to the planes of the base faces (2, 3), the distance between at least a first end (7, 8) of the tab and the axis of rotation (6) being substantially greater than the distance between at least one of the two base faces (2, 3) and said axis of rotation, said means (5) comprising a rotary shaft (20) secured to the tab (4) and an orifice (21) made in the spacer (1), being centered on the axis of rotation (6), the shaft (20) being mounted to turn in the orifice, the cage being **characterized by** the fact that it further comprises:
· means (26) for preventing the shaft (20) from moving in translation relative to the spacer (1); and
· means (22) for applying a resilient force to the tab (4) tending to press the tab against the side wall of the spacer, said means being constituted by the fact that the shaft (20) is mounted in the orifice (21) under elastic traction.

2. A cage according to claim 1, **characterized by** the fact that the first end (7, 8) of the tab (4) has a chamfered shape.

3. A cage according to claim 1 or claim 2, **characterized by** the fact that it includes a slot (31) made in the face (32) of the tab (4) which faces towards the spacer (1).

4. A cage according to any one of claims 1 to 3, **characterized by** the fact that it includes means (10) for blocking the tab (4) when it is brought into a position that is substantially orthogonal to the planes of the base faces (2, 3).

5. A cage according to claim 4, **characterized by** the fact that the means (10) for blocking the tab (4) when it is brought into a position substantially orthogonal to the planes of the base faces (2, 3) comprise at least one groove (30) made in the side wall (13) of the spacer (1) and opening out into the two base faces (2, 3), the groove (30) being made in the side wall of the spacer so as to received the tab (4) when the tab is substantially orthogonal to the planes of the base faces (2, 3), the width of the groove (30) being substantially equal to the width of the tab.

6. A cage according to any one of claims 1 to 5, **characterized by** the fact that the first end (7, 8) of the tab carries a set of teeth (11).

7. A cage according to any one of claims 1 to 6, **characterized by** the fact that the respective distances between each of the two ends (7, 8) of the tab (4) and the axis of rotation (6) are greater than the respective distances between each of the two base faces (2, 3) and said axis of rotation.

8. A cage according to any one of claims 1 to 7, **characterized by** the fact that said spacer (1) includes a cavity (40) open over at least one of the two base faces (2, 3), said cavity being suitable for containing a bone graft (41).

9. A cage according to any one of claims 1 to 8, **characterized by** the fact that the disk is substantially in the form of a rectangular parallelepiped.

10. A cage according to claim 9, **characterized by** the fact that the tab (4) is mounted to turn on one (9) of the two small side faces of the spacer (1), the axis of rotation (6) being substantially perpendicular to said small side face.

11. A cage according to claim 10, **characterized by** the fact that the width and the length of the tab (4) are substantially equal respectively to the width and to the length of the small side face (9) on which it is mounted to turn.
